# EUROPÄISCHE PATENTANMELDUNG

(11) **EP 3 838 245 A1**
(43) Veröffentlichungstag der Anmeldung: **23.06.2021**
(21) Anmeldenummer: 19216765.8
(22) Anmeldetag: 17.12.2019
(51) Int. Cl.: A61G 7/08, A61B 6/00

(54) **BASISMODUL, MEDIZINISCHES SYSTEM UND VERFAHREN ZUM BETRIEB DES MEDIZINISCHEN SYSTEMS**

(71) Anmelder: Siemens Healthcare GmbH, 91052 Erlangen (DE)
(72) Erfinder: Deuringer, Josef, 91074 Herzogenaurach (DE); Dietz, Volker, 91315 Höchstadt an der Aisch (DE); Dirauf, Franz, 96231 Bad Staffelstein (DE); Garcia, Elmar, 90427 Nürnberg (DE)

(57) **Zusammenfassung**

Ein Basismodul (10) für ein mobiles medizinisches System (1), welches dazu ausgebildet ist, Wegstrecken autonom oder semiautonom zurückzulegen, umfasst ein mehrere Abrollkörper (18, 18A, 18B, 18C, 18D, 18E, 18F, 18G, 18H) aufweisendes Fahrwerk (12). Die Abrollkörper (18, 18A, 18B, 18C, 18D, 18E, 18F, 18G, 18H) sind um Rotationsachsen (R) drehbar gelagert und mittels Verstelleinrichtungen (15) in vertikaler Richtung (V) derart verstellbar, dass jeder Abrollkörper (18, 18A, 18B, 18C, 18D, 18E, 18F, 18G, 18H) zwischen einer ersten Position, in der der jeweilige Abrollkörper (18, 18A, 18B, 18C, 18D, 18E, 18F, 18G, 18H) in Kontakt zu einer Bodenfläche (B) steht, und einer zweiten Position, in der der jeweilige Abrollkörper (18, 18A, 18B, 18C, 18D, 18E, 18F, 18G, 18H) von der Bodenfläche (B) beabstandet ist, bewegbar ist. Die Erfindung betrifft ferner ein medizinisches System (1) mit einem derartigen Basismodul (10) und ein Verfahren zum Betrieb des medizinischen Systems (1).

## Beschreibung

Die vorliegende Erfindung betrifft ein Basismodul für ein mobiles medizinisches System, das zur autonomen und/oder semi-autonomen Fortbewegung ausgebildet ist. Die vorliegende Erfindung betrifft ferner das zum autonomen oder semi-autonomen Zurücklegen von Wegstrecken ausgebildete medizinische System und ein Verfahren zum Betrieb eines derartigen mobilen medizinischen Systems. Das medizinische System stellt eine Plattform allgemeiner Art dar, die insbesondere als Basis zum Transport von Bildgebungseinrichtungen, medizinischer Ausrichtung und/oder Lasten, insbesondere in einer klinischen Umgebung, dient.

Der Megatrend der Elektrifizierung, Automatisierung und Digitalisierung hält nicht nur im Bereich der Industrie Einzug (Stichwort: Industrie 4.0), sondern spiegelt sich auch bei der Weiterentwicklung von medizinischen Abläufen, klinischen Verfahren und Medizingeräten, insbesondere von bildgebenden Einrichtungen, wieder. Diese Entwicklung wird allgemein mit den Schlagworten Digital Hospital oder Hospital 2.0 bezeichnet.

Im Bereich der Medizintechnik sind mobile medizinische Systeme, beispielsweise mobile Bildgebungsanlagen, bekannt, die dazu ausgebildet sind, Wege bzw. Wegstrecken, beispielsweise innerhalb eines Krankenhauses, autonom oder teil-autonom zurückzulegen. So ist beispielsweise aus US 2018/0242932 A1 eine autonom bzw. teil-autonom bewegbare bzw. verfahrbare Bildgebungsanlage bekannt.

Neben derartigen mobilen medizinischen Systemen werden u. a. Maßnahmen vorgeschlagen, die beispielsweise die Optimierung und Digitalisierung von bestehenden Steuerungsabläufen für solche medizinischen Systeme betreffen. So beschreibt DE 10 2014 208 463 A1 beispielsweise ein als mobile Patientenliege ausgeführtes mobiles medizinisches System und ein Verfahren zum Steuern einer derartigen Patientenliege.

Der Großteil der in Krankenhäusern, Medizinzentren und Praxen installierten medizinischen Systeme ist zum gegenwärtigen Zeitpunkt tatsächlich noch nicht dazu ausgebildet, Wegstrecken autonom oder teil-autonom zurückzulegen. Selbst robotergestützte medizinische Systeme sind überwiegend fest installiert, wobei diese trotz der festen Installation ein hohes Maß an Freiheitsgraden der gesteuerten Bewegung bieten können. DE 10 2011 083 876 B4 offenbart beispielsweise eine stationäre Bildgebungsanlage, die ein hochbewegliches Röntgensystem umfasst.

DE 10 2014 115 901 A1 beschreibt eine Bodenplattform für Operationstische, die eine Schnittstelle zu Befestigung einer Patientenlagereinheit zur Lagerung eines Patienten sowie eine omnidirektionale, elektrische Antriebseinheit umfasst. Die Antriebseinheit ist derart ausgebildet, dass die Bodenplattform allein durch die Antriebseinheit innerhalb einer vorbestimmten Ebene in jede beliebige Richtung verfahrbar und drehbar ist. Ferner ist eine Bedieneinheit zum Steuern der Antriebseinheit vorgesehen, wobei diese ein manuelles Betätigungselement umfasst. Die Bedieneinheit weist zudem eine Steuereinheit auf, die in Abhängigkeit der Betätigung des Betätigungselementes Ansteuerungssignale für die Antriebseinheit ermittelt und an die Antriebseinheit überträgt. Auf Grundlage der Ansteuerungssignale bewegt die Antriebseinheit die Bodenplattform. Die Antriebseinheit des beschriebenen medizinischen Systems umfasst mehrere unabhängig voneinander ansteuerbare, angetriebene Räder, wobei die Steuereinheit durch die individuelle Ansteuerung der angetriebenen Räder die Bewegungsrichtung der Bodenplattform steuert.

Das medizinische System der DE 10 2014 115 901 A1 ist sehr aufwendig und kostspielig zu realisieren, da es acht komplexe Mecanum-Räder aufweist. Als Mecanum-Rad wird hierbei ein Allseitenrad (auch: omnidirektionales Rad) bezeichnet, bei der die Lauffläche des Hauptades von Rollen gebildet wird, deren Achsen schräg bezüglich der tangentialen Richtung des Hauptrades angeordnet sind. Da mehrere Mecanum-Räder verbaut werden, ist das medizinische System störanfällig und es in der Regel von einem erhöhten Wartungsbedarf auszugehen. Das medizinische System ist zudem nicht universell nutzbar, sondern lediglich zur Befestigung einer Patientenlagereinheit ausgebildet.

Aus US 2018/0242932 A1 ist ein mobiles medizinisches System bekannt, das zwei verschiedene Antriebsmechanismen aufweist. Zum einen ist ein so genannter Präzisionsantrieb (engl.: fine movement mechanism) vorgesehen, der beispielsweise mehrere angetriebene Räder umfasst. Zum anderen ist ein so genannter Grobantrieb (engl.: gross movement mechanism) vorgesehen, der mehrere, ebenfalls omnidirektionale Radbaugruppen umfasst, deren Bauart im Wesentlichen derjenigen der Mecanum-Räder entspricht. Als eine zu den mehreren angetriebenen Rädern alternative Antriebsart wird ein so genannter Riemenantrieb vorgeschlagen. Die Verwendung zweier unterschiedlich ausgeführter Antriebssysteme in einem mobilen medizinischen System ist aufwendig, teuer und in der Regel störanfällig und bringt somit einen erhöhten Wartungsbedarf mit sich. Das vorgeschlagene medizinische System ist zudem nicht universell nutzbar, sondern lediglich für eine mobile Bildgebungseinrichtung vorgesehen.

Handelsübliche medizinische Bildgebungseinrichtungen bzw. Bildgebungsmodalitäten oder Patientenliegen sind im Allgemeinen nicht zur autonomen oder teil-autonomen Fortbewegung ausgebildet, sondern müssen vielmehr von einem Benutzer manuell an den jeweiligen Einsatzort gebracht werden. Während die Person das mobile Gerät händisch verfährt, ist Arbeitskraft gebunden, insbesondere können von dieser Person keine anderen, insbesondere keine medizinische relevante, Aufgaben wahrgenommen werden.

Es ist Aufgabe der vorliegenden Erfindung, ein verbessertes medizinisches System, welches zum autonomen oder semi-autonomen Zurücklegen von Wegstrecken ausgebildet ist, anzugeben. Es ist insbesondere Aufgabe der vorliegenden Erfindung, ein Basismodul mit einem Fahrwerk für ein derartiges medizinisches System und ein Verbessertes Verfahren zum Betrieb eines derartigen medizinischen Systems anzugeben.

Gemäß der Erfindung wird diese Aufgabe gelöst durch ein Basismodul mit den Merkmalen des Anspruchs 1, durch ein medizinisches System mit den Merkmalen des Anspruchs 11 und ein Verfahren zum Betrieb des medizinischen Systems mit den Merkmalen des Anspruchs 15.

Vorteilhafte Ausgestaltungen der Erfindung sind Gegenstand der Unteransprüche.

Ein Basismodul für ein mobiles medizinisches System, welches dazu ausgebildet ist, Wegstrecken autonom oder semi-autonom zurückzulegen, umfasst ein mehrere Abrollkörper aufweisendes Fahrwerk. Die Abrollkörper sind um Rotationsachsen drehbar gelagert und mittels Verstelleinrichtungen in vertikaler Richtung derart verstellbar, dass jeder Abrollkörper zwischen einer ersten Position, in der der jeweilige Abrollkörper in Kontakt zu einer Bodenfläche steht, und einer zweiten Position, in der der jeweilige Abrollkörper von der Bodenfläche beabstandet ist, bewegbar ist.

Die vorliegende Erfindung stellt eine alternative, verhältnismäßig einfach aufgebaute und modular ausgestaltete Baukastenlösung für ein autonom oder teil-autonom (auch: semi-autonom) bewegbares bzw. verfahrbares medizinisches System bereit. Das vorstehend genannte Basismodul dient zur Fortbewegung des modular aufgebauten medizinischen Systems. Durch die Verstellbarkeit der Abrollkörper in vertikaler Richtung können auf einfache Weise verschiedene Fahrmanöver, insbesondere auf engem Raum, durchgeführt werden. Abrollkörper, die die Durchführung des jeweiligen Fahrmanövers behindern oder beeinträchtigen würden, werden entsprechend automatisch eingezogen, so dass diese keinen Kontakt mehr zur Bodenfläche haben. Auf diese Weise kann auf mechanisch komplexe Bauteile, wie etwa omnidirektionale Räder bzw. Mecanum-Räder, verzichtet werden.

Das erfindungsgemäße medizinische System kann beispielsweise als mobile Plattform für Bildgebungseinrichtungen, wie etwa C-Bogenröntgengeräte, Computertomographen, Ultraschalleinrichtungen, und/oder für medizinische Ausrüstung, Patientenliegen oder Ähnliches Anwendung finden. Das medizinische System kann in möglichen Ausgestaltungen eine kosteneffiziente Variante einer mobilen Plattform für Medizincontainer oder Ähnliches darstellen, die modular und bedarfsorientiert, d.h. je nach momentanem Einsatz, bestückt werden. Das medizinische System ist somit vielfältig einsetzbar.

Das medizinische System ist vorwiegend für den Einsatz in einer klinischen Umgebung (Krankenhaus, Medical Center, Praxis, etc.) vorgesehen und im Sinne des modularen Baukastenprinzips für verschiedenste Anwendungen einsetzbar, beispielsweise für Bildgebungseinrichtungen, wie etwa C-Bögen, Computertomographen oder Ultraschalleinrichtungen, und/oder für medizinische Ausrüstung, Patientenliegen oder Ähnliches. In anderen Ausgestaltungen dient das medizinische System beispielsweise als mobiler Trolley. Zusammenfassend gesprochen stellt das medizinische System eine verfahrbare bzw. bewegbare, respektive hinsichtlich ihres Ortes verlagerbare, Plattform dar, um Anordnungen mit größerer Masse (insbesondere bildgebende Modalitäten, aber auch andere Arten von schweren mobilen klinischen Tischen, Schränken oder Aufbewahrungsbehältnissen) autonom oder zumindest teil-autonom zwischen zumindest zwei Orten zu bewegen.

In Ausgestaltungen sind zumindest zwei Rotationsachsen der Abrollkörper zueinander in einer Anordnung angeordnet, die von einer parallelen oder antiparallelen Anordnung abweicht. Mit anderen Worten weist das Fahrwerk des Basismoduls Abrollkörper mit zumindest zwei verschiedenen Rotationachsen auf, die zueinander in einem Winkel, der von null verschieden ist, orientiert sind.

In Ausgestaltungen sind zumindest zwei Rotationsachsen der Abrollkörper zueinander in einem stumpfen, insbesondere rechten, Winkel angeordnet. Das Fahrwerk des Basismoduls weist insbesondere unterschiedlich ausgerichtete Abrollkörper auf, die beispielsweise der Vorwärts- bzw. Rückwärtsbewegung des medizinischen Systems und einer seitlichen Bewegung des medizinischen Systems dienen.

In Ausgestaltungen bilden die Rotationsachsen der Abrollkörper des Fahrwerks zumindest eine umlaufende, insbesondere rechteckige oder quadratische, Anordnung. Derartige Anordnungen sind mechanisch sehr stabil.

In Ausgestaltungen sind die Abrollkörper jeweils paarweise um die Rotationsachsen drehbar gelagert. Mit anderen Worten sind um jede Rotationsachse zumindest zwei Abrollkörper konzentrisch angeordnet, um die Stabilität des Fahrwerks zu verbessern. Auf diese Weise wird eine hinreichende Stabilität auch dann sichergestellt, wenn beispielsweise ein Abrollkörper zur Durchführung eines bestimmten Fahrmanövers eingezogen werden muss und somit keinen Kontakt mehr zur Bodenfläche hat.

In Ausgestaltungen ist die Verstelleinrichtung als einstellbarer Stoßdämpfer, insbesondere Gasdruckstoßdämpfer, ausgeführt.

In Ausgestaltungen sind die Abrollkörper im Wesentlich zylindrisch ausgeführt. Die Abrollkörper weisen beispielsweise einen im Wesentlichen konstanten Durchmesser auf. Alternativ dazu weisen die Abrollkörper in Ausgestaltungen eine Bombierung auf, insbesondere derart, dass sich der Durchmesser des Abrollkörpers endseitig verjüngt.

In Ausgestaltungen weist das Basismodul eine Vibrationssensorik zur Erfassung von Vibrationen, insbesondere von während der Fahrt auftretenden Vibrationen, auf. Die Vibrationssensorik weist beispielsweise geeignet ausgebildete Beschleunigungssensoren zur Erfassung der Vibrationen auf.

In Ausgestaltungen weist das Basismodul eine Beschleunigungssensorik zur Erfassung von Beschleunigungen, insbesondere Kollisionen, auf. Die Beschleunigungssensorik weist beispielsweise geeignet ausgebildete Beschleunigungssensoren, insbesondere zur Erfassung von Kollisionen mit bewegten oder unbewegten Objekten, auf.

Die Erfindung betrifft ferner das vorstehend bereits genannte medizinische System. Das Medizinisches System ist dazu ausgebildet, Wegstrecken autonom oder semi-autonom zurückzulegen und umfasst
- das bereits beschriebene Basismodul,
- ein Umgebungserfassungsmodul, welches eine zur Erfassung der Umgebung des medizinischen Systems ausgebildete Umgebungssensorik aufweist und
- ein Steuerungsmodul zur Steuerung der Fortbewegung des Basismoduls unter Berücksichtigung der mittels des Umgebungserfassungsmoduls sensorisch erfassten Umgebung des medizinischen System.

Die autonome oder zumindest teil-autonome Bewegung wird dadurch realisiert, dass das medizinische System eine Trägerplattform für mehrere zusammenschaltbare Module (auch: Baukastenmodule) darstellt. Das medizinische System umfasst in Ausgestaltungen ein Antriebsmodul, welches durch das vorstehend beschriebene Basismodul implementiert ist. Das Basismodul dient zumindest überwiegend für die rein mechanische Fortbewegung des medizinischen Systems und umfasst eine relativ einfache und robuste Mechanik, welche eine Fortbewegung des medizinischen Systems auf konstruktiv einfach gestalteten Abrollkörpern ermöglicht.

Das medizinische System weist ferner das Umgebungserfassungsmodul auf, welches in Ausgestaltungen mehrere Geber/Fühler und beispielsweise weitere, verschiedenartige Sensorelemente (z.B. Video/Kamera, LIDAR, Nahfeldradar, Weitfeldradar, etc.) umfasst und zum Erfassen des Umfelds des medizinischen Systems ausgebildet sind.

Das medizinische System weist ferner das Steuerungsmodul auf, welches dazu vorgesehen ist, Informationen aus dem Umgebungserfassungsmodul intern zu verarbeiten und/oder zu qualifizieren. Das Steuerungsmodul generiert beispielsweise in Abhängigkeit dieser Informationen Fahrsteuerungsbefehle, die es an das Basismodul zur Steuerung der Fortbewegung des medizinischen Systems weiterleitet.

Um eine Autonomie bzw. Autarkie von etwaig vorhandenen elektrischen Versorgungsnetzen zu erhalten, umfasst das medizinische System in Ausgestaltungen optional ein Energiespeichermodul, welches die Strom- und Spannungsversorgung für die bereits genannten Baukastenmodule zur Verfügung stellt.

Das Basismodul, das Umgebungserfassungsmodul, das gegebenenfalls optional vorgesehene Energiespeichermodul und das Steuerungsmodul werden im Folgenden zusammenfassend als Module (auch: Baukastenmodul) des medizinischen Systems bezeichnet. Diese Module sind in Ausgestaltungen interkommunikations- und netzwerkfähig ausgeführt. Hierzu sind die Module beispielsweise mit geeigneten Schnittstellen versehen, die zur drahtlosen oder drahtgebundenen Kommunikation, insbesondere in Echtzeit, ausgebildet sind. Die Schnittstellen umfassen beispielsweise ein internes Bussystem, das insbesondere durch ein Ethernet, einen CAN-Bus oder durch ein anderes für medizinische Anwendungen geeignetes Feldbussystem realisiert ist. Das Ethernet, der CAN-Bus und/oder das Feldbussystem ist in Ausgestaltungen echtzeitfähig, so dass die Module miteinander in Echtzeitkommunikation treten können. Die Module weisen in Ausgestaltungen ein oder mehrere Microchips, Prozessoren, Controller und/oder anderweitige Elektronikkomponenten auf, die zur Durchführung von digitalen Operationen ausgebildet sind. Die Module weisen in Ausgestaltungen ferner Speicher, insbesondere nicht-flüchtige Speicher, und Arbeitsspeicher, insbesondere flüchtige Arbeitsspeicher, auf. Auf allen Modulen 10, 20, 30, 40 sind in Ausgestaltungen Betriebssysteme (engl.: operating system, OS) und/oder weitere Softwarepakete, insbesondere in den jeweilig nicht-flüchtigen Speichern, installiert. Die Module sind in Ausgestaltungen eigendiagnosefähig und beispielsweise dafür eingerichtet, Zustände über ihre Funktionsweise abzulegen und insbesondere im Rahmen einer Ferndiagnose/Fernwartung an eine Auswerteeinheit oder Basisstation eines externen Netzwerks zu übermitteln. Das externe Netzwerk ist beispielsweise ein drahtgebundenes Netzwerk oder ein Drahtlosnetzwerk oder weist sowohl drahtgebundenen Subnetze als auch drahtlose Subnetze auf. Das interne Netzwerk bzw. Bussystem weist beispielsweise einen für Ferndiagnose und/oder Fernwartung eingerichteten Zugangsport in eine Cloud-Anwendung auf.

In Ausgestaltungen erfolgt die Versorgung des medizinischen Systems mit Energie mittels des vorstehend bereist genannten Energiespeichermoduls. Das Steuerungsmodul ist vorzugsweise dazu ausgebildet, die Fortbewegung des Basismoduls unter Berücksichtigung des Energie- bzw. Ladezustands des Energiespeichermoduls zu steuern.

In Ausgestaltungen weist das Steuerungsmodul eine Benutzerschnittstelle auf und ist zur Sprachsteuerung und/oder Gestensteuerung ausgebildet. Die Benutzerschnittstelle weist vorzugsweise entsprechend ausgestaltete Erfassungsgeräte, wie etwa Mikrofone, optische Sensoren und/oder Kameras auf. Im Steuerungsmodul ist vorzugsweise eine Software zur Spracherkennung und/oder zur Gestenerkennung implementiert.

In Ausgestaltungen ist das medizinische System dazu ausgebildet, eine Bildgebungseinrichtung, medizinische Ausrüstung, eine Patientenliege und/oder anderweitige Lasten zu tragen und fortzubewegen. Derartige Komponenten können beispielsweise an einer Trägerplattform des medizinischen Systems lösbar befestigt oder fest installiert sein.

Bei einem Verfahren zum Betrieb des vorstehend beschriebenen medizinischen Systems generiert das Steuerungsmodul zur Steuerung der Fortbewegung Fahrsteuerbefehle und übermittelt diese zur Ausführung an das Basismodul. Die Ausführung zumindest eines der Fahrsteuerbefehle durch das Basismodul beinhaltet das Verstellen zumindest eines der Abrollkörper in vertikaler Richtung. Auf diese Weise können Abrollkörper, die die Durchführung des entsprechenden Fahrmanövers behindern würden, in vertikaler Richtung so verstellt werden, dass diese keinen Kontakt mehr zur Bodenfläche, auf der sich das medizinische System fortbewegt, haben. Das Verstellen der jeweiligen Abrollkörper in vertikaler Richtung erfolgt in Abhängigkeit des auszuführenden Fahrmanövers bevorzugt automatisch.

In Ausgestaltungen erfolgt die Generierung der Fahrsteuerbefehle in Abhängigkeit von Daten einer Vibrationssensorik, die insbesondere indikativ für während der Fahrt auftretende Vibrationen sind. Die Daten des Vibrationssensorik stellen somit einen Indikator für Erschütterungen und Vibrationen dar und/oder enthalten Informationen über während der Fahrt auftretende Vibrationen. Dies lässt insbesondere Rückschlüsse über die Beschaffenheit der Bodenfläche zu, auf der sich das medizinische System fortbewegt.

In Ausgestaltungen erfolgt die Generierung der Fahrsteuerbefehle in Abhängigkeit von Daten einer Beschleunigungssensorik, die insbesondere indikativ für während der Fahrt auftretende Beschleunigungen sind. Die Daten des Beschleunigungssensorik stellen beispielsweise einen Indikator für möglicherweise aufgetretene Kollisionen mit bewegten oder unbewegten Objekten dar und können insbesondere dazu verwendet werden, um einen automatischen Nothalt des medizinischen Systems zu implementieren.

In Ausgestaltungen erfolgt die Generierung der Fahrsteuerbefehle in Abhängigkeit von qualifizierten Umgebungsdaten, die insbesondere indikativ für das Umfeld des medizinischen Systems sind. Die qualifizierten Umgebungsdaten stellen beispielsweise einen Indikator für in der mittelbaren und/oder unmittelbaren Umgebung des medizinischen Systems vorhandene Objekte. Die Berücksichtigung der qualifizierten Umgebungsdaten ist für die Wegplanung maßgeblich und ermöglicht insbesondere das autonome bzw. semi-autonome Planen und Zurücklegen von Wegstrecken, insbesondere innerhalb einer klinischen Umgebung oder einer Feldumgebung.

In Ausgestaltungen erfolgt die Generierung der Fahrsteuerbefehle in Abhängigkeit von Diagnoseprotokollen eines Batteriemanagementsystems, die insbesondere indikativ für einen Ladezustand und/oder einen Verfügbarkeitszustand eines Energiespeichers sind. Derartige Diagnoseprotokolle können insbesondere dazu genutzt werden, die Einsatzfähigkeit des medizinischen Systems vorab zu beurteilen.

Für eine weitere Beschreibung der Erfindung wird auf das in den Zeichnungsfiguren gezeigte Ausführungsbeispiel verwiesen. Es zeigen in einer schematischen Darstellung:
- Fig. 1:: den modularen Aufbau eines mobilen medizinischen Systems, das zum autonomen oder semi-autonomen Zurücklegen von Wegstrecken ausgebildet ist;
- Fig. 2:: das Zusammenwirken der Module bei einem Verfahren zum Betrieb des mobilen medizinischen Systems der Fig. 1;
- Fig. 3: ein mobiles medizinisches System mit einer Bildgebungseinrichtung gemäß einem möglichen Ausführungsbeispiel der Erfindung;
- Fig. 4: ein mobiles medizinisches System mit einer Bildgebungseinrichtung gemäß einem weiteren möglichen Ausführungsbeispiel der Erfindung;
- Fig. 5: ein mobiles medizinisches System mit einer Patientenliege gemäß einem weiteren möglichen Ausführungsbeispiel der Erfindung;
- Fig. 6: ein Umgebungserfassungsmodul des mobilen medizinischen Systems in einer möglichen Ausgestaltung der Erfindung;
- Fig. 7: ein Energiespeichermodul des mobilen medizinischen Systems in einer möglichen Ausgestaltung der Erfindung.

Identische oder einander entsprechende Teile oder Komponenten sind in allen Figuren mit den gleichen Bezugszeichen versehen.

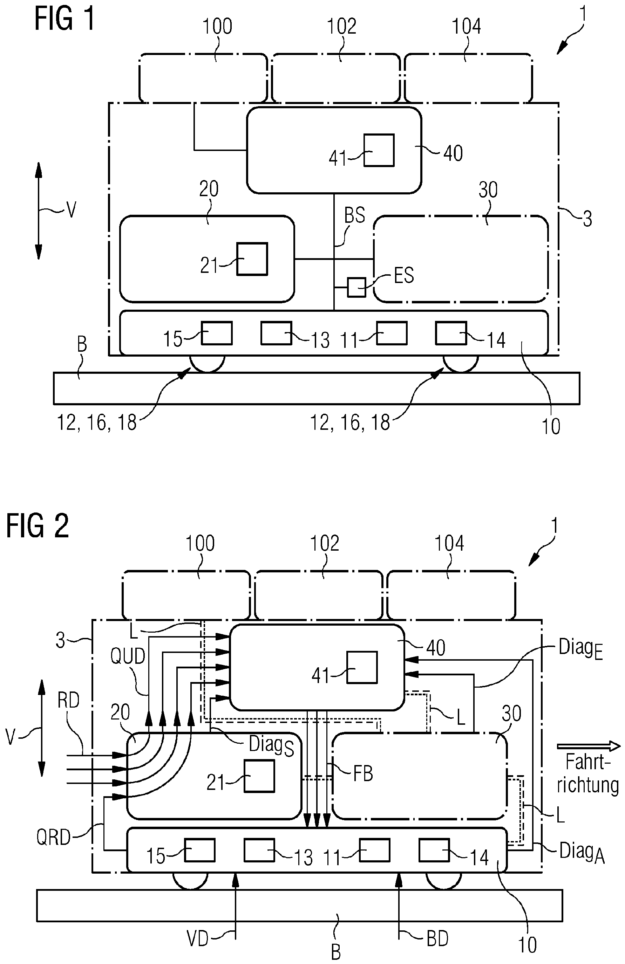

Figur 1 zeigt schematisch eine zum autonomen oder zumindest teil-autonomen Zurücklegen von Wegstrecken ausgebildetes mobiles medizinisches System 1 in einer schematischen Darstellung. Das medizinische System 1 ist modular aufgebaut und umfasst eine in Figur 1 gestrichelt dargestellte Trägerplattform 3 für mehrere netzwerk- und kommunikationsfähige Module 10, 20, 30, 40, die zum gegenseitigen Austausch von Daten mit entsprechend kabelgebundenen oder kabellosen Schnittstellen ausgebildet sind.

Die Trägerplattform 3 stellt die Basis für ein Baukasten- bzw. Modulkonzept dar, bei dem beispielsweise in einem Innenbereich der Trägerplattform 3 mehrere unterschiedliche Module 10, 20, 30, 40 angeordnet und zusammengeschaltet werden, so dass diese funktional miteinander kommunizieren können. Figur 1 zeigt dabei lediglich ein nichteinschränkend aufzufassendes Ausführungsbeispiel dieses Baukasten- bzw. Modulkonzepts, d. h. Anzahl und Typ der Module 10, 20, 30, 40, mit denen die Trägerplattform 3 bestückt wird, wird im Allgemeinen "on demand", d. h. bedarfsorientiert je nach gewünschtem Einsatzgebiet, angepasst.

Bei den Modulen 10, 20, 30, 40 handelt es sich insbesondere um ein Basismodul 10, ein Umgebungserfassungsmodul 20, ein gegebenenfalls optional vorgesehenes Energiespeichermodul 30 und ein Steuerungsmodul 40.

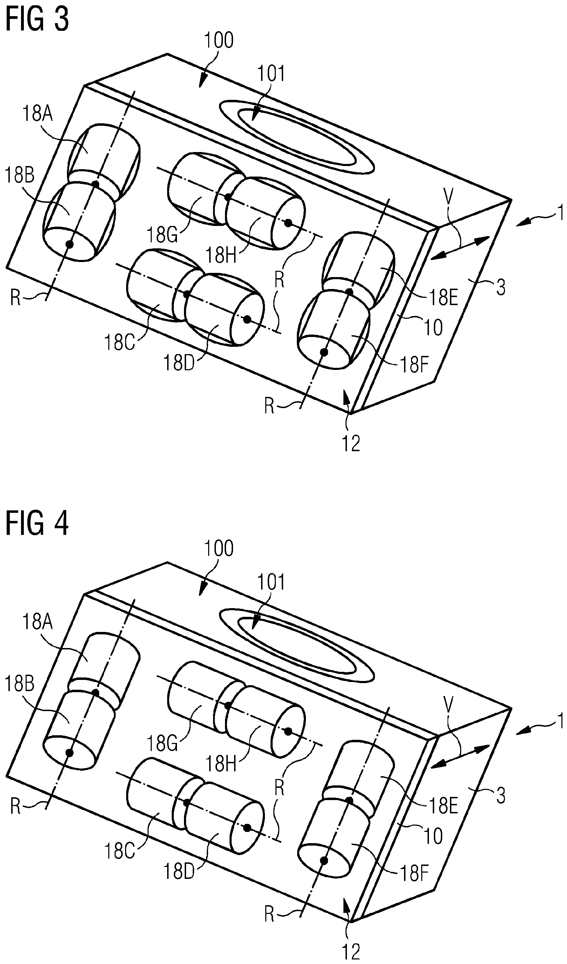

Nähe des Bodens befindet sich das Basismodul 10 mit einem Fahrwerk 12 und einem, beispielsweise elektrischen, Antrieb 14. Das Fahrwerk 12 umfasst mehrere motorisch angetriebene oder antreibbare Antriebsmittel 16, die als im Wesentlichen zylindrische bzw. tonnenförmige Abrollkörper 18 ausgeführt sind. Wie insbesondere in Figur 3 dargestellt, ist der Durchmesser der Abrollkörper 18 in vorteilhaften Ausgestaltungen nicht konstant, sondern weist eine Bombierung dergestalt auf, dass der Durchmesser in Richtung zu den axialen Enden des jeweiligen Abrollkörpers 18 hin zumindest geringfügig abnimmt.

Die Abrollkörper 18 sind beispielsweise mit einer Gummi-, Linoleum,- oder Kunststoffoberfläche versehen. Linoleum ist in vorteilhafter Weise antistatisch, leicht fungizid und bakteriostatisch, d.h. es hemmt ein etwaiges Bakterienwachstum. Ursache ist die permanente Emission geringer Mengen verschiedener Aldehyde, die aus der so genannten Leinölautoxidation an Luft oder aus der Oxidationsreaktion im Herstellungsprozess stammen. Aus diesem Grund wird Linoleum häufig als Bodenbelag in Gebäuden mit erhöhten Hygieneanforderungen, wie etwa Krankenhäusern oder Arztpraxen, verwendet.

Die tonnenförmigen Abrollkörper 18 können in Ausgestaltungen eine Edelstahlkonstruktion aufweisen, welche zur Aufnahme einer Walze, beispielsweise aus Vollgummi, ausgebildet ist. In anderen Ausführungen ist die Edelstahlkonstruktion so ausgeführt, dass in diese Kunststoffrollen einsetzbar sind. Die Kunststoffrollen weisen beispielsweise einen thermoplastischen Polyurethan-Laufbelag auf. Dies erleichtert die Mobilität bzw. die Verfahrbarkeit der Trägerplattform 3 bzw. des medizinischen Systems 1.

Die Antriebsmittel 16 sind in einer vergleichsweise einfachen Antriebsmechanik angeordnet, welche eine Fortbewegung auf den Abrollkörpern 18 ermöglicht.

Ecken und Kanten der Abrollkörper 18 sind in Ausgestaltungen in vorteilhafter Weise abgerundet ausgebildet. Die einfache geometrische Formgebung der im Wesentlichen tonnen- bzw. zylinderförmigen Abrollkörper 18 ermöglicht eine einfache Reinigung, insbesondere mittels gängiger Desinfektionschemikalien.

Die Trägerplattform 3 ist beispielsweise dazu ausgebildet, eine Bildgebungseinrichtung 100 (auch: Bildgebungsmodalität), weitere medizinische Ausrüstung 102 und/oder zusätzliche medizinische Lasten 104 und/oder weitere Behältnisse bzw. Container zu tragen. Diese Komponenten sind beispielswiese mittels Spann- und/oder Clip-Schnellverschlüssen an der Trägerplattform 3 befestigbar.

In Ausgestaltungen, bei denen keine Bildgebungseinrichtung 100 an der Trägerplattform 3 des modularen medizinischen Systems 1 angebracht ist, kann das medizinische System 1 beispielsweise zum Transport von Containern eingesetzt werden, insbesondere um mit Körperflüssigkeiten kontaminierte Chirurgie- und/oder Operationsprodukte oder Hilfsmittel zu transportieren. Die Container sind beispielsweise mittels Spann- und/oder Clip-Schnellverschlüssen an der Trägerplattform 3 lösbar befestigbar.

Das mobile medizinische System 1 umfasst ferner das Umgebungserfassungsmodul 20 zum Erfassen der Umgebung, insbesondere der stationären Umgebung und von stationärer und/oder bewegter Objekte im Umfeld des medizinischen System 1. Das Umgebungserfassungsmodul 20 dient ferner zur Planung des Fahrweges. Das optionale Energiespeichermodul 30 dient zur Versorgung der Module 10, 20, 30, 40 mit Energie, insbesondere elektrischer Energie, und das Steuerungsmodul 40 zur Steuerung und/oder Regelung der Fortbewegung unter Berücksichtigung der, insbesondere sensorisch erfassten, Umgebung des medizinischen System 1 und des Energie- bzw. Ladezustands des Energiespeichermoduls 30.

Die Module 10, 20, 30, 40 sind interkommunikations- und netzwerkfähig ausgeführt. Hierzu sind die Module 10, 20, 30, 40 mit geeigneten Schnittstellen versehen, die zur drahtlosen oder drahtgebundenen Kommunikation, insbesondere in Echtzeit, ausgebildet sind. Diese Schnittstellen umfassen beispielsweise ein internes Bussystem BS, das insbesondere durch ein Ethernet, einen CAN-Bus oder über ein anderes für medizinische Anwendungen geeignetes Feldbussystem realisiert ist. Das Ethernet, der CAN-Bus und/oder das Feldbussystem ist in Ausgestaltungen echtzeitfähig, so dass die Module 10, 20, 30, 40 miteinander in Echtzeitkommunikation treten können.

Die Module 10, 20, 30, 40 weisen ein oder mehrere Microchips, Prozessoren, Controller und/oder anderweitige Elektronikkomponenten auf, die zur Durchführung von digitalen Datenoperationen ausgebildet sind. Die Module 10, 20, 30, 40 weisen ferner Speicher, insbesondere nicht-flüchtige Speicher, und Arbeitsspeicher, insbesondere flüchtige Arbeitsspeicher, auf. Auf allen Modulen 10, 20, 30, 40 sind in Ausgestaltungen Betriebssysteme (engl.: operating system, OS) und/oder weitere Softwarepakete, insbesondere in den jeweilig nicht-flüchtigen Speicher, installiert.

Ähnlich der Bordnetztopologie eines modernen Kraftfahrzeugs sind die Module 10, 20, 30, 40 in Ausgestaltungen dazu ausgebildet, Protokolle austauschen, also insbesondere zu senden und/oder zu empfangen. Die Module 10, 20, 30, 40 sind in Ausgestaltungen als elektronische Steuergeräte ausgestaltet oder umfassen ein oder mehrere elektronische Steuergeräte. Die Steuergeräte sind beispielsweise in den Modulen 10, 20, 30, 40 eingebettet.

In Ausgestaltungen erfolgt die Kommunikation der Module 10, 20, 30, 40 untereinander mittels eines sicheren, echtzeitfähigen Bussystems BS, welches Diagnoseprotokolle unterstützt und zusätzlich für die drahtgebundene oder drahtlose Kommunikation mit einem weiteren externen Netzwerk eingerichtet ist.

Die Module 10, 20, 30, 40 sind beispielsweise eigendiagnosefähig und somit dafür eingerichtet, Zustände über ihre Funktionsweise (beispielsweise Fehlercodes oder Fehlerspeichereinträge) im jeweiligen Speicher oder in einem modulinternen Fehlerspeicher oder in einem zum Ablegen von Fehlern vorgesehenen Speicherbereich abzulegen.

Die Fehlercodes oder Fehlerspeichereinträge sind über Diagnoseprogramme bzw. so genannte Diagnose-Services auslesbar, insbesondere mittels eines so genannten Diagnostic Scan Tools. Insbesondere kann ein so genanntes "P-Code" Tool eingesetzt werden, welches beispielsweise im Rahmen der On-Board-Diagnose (OBD) der Automobilindustrie angewendet wird.

Die Fehlercodes oder Fehlerspeichereinträge sind mit Hilfe einer externen Auswerteeinheit, wie etwa einem Laptop oder Notebook, auslesbar. Die Fehlercodes oder Fehlerspeichereinträge können, beispielsweise als Antwort auf einen externen Trigger, der etwa im Rahmen einer Ferndiagnose und/oder einer Fernwartung an das jeweilige Modul 10, 20, 30, 40 übertragen wird, an die externe Auswerteeinheit oder an eine Basisstation, die in einem externen Netzwerk angeordnet ist, drahtlos oder drahtgebunden übermittelt werden. Der externe Trigger ist beispielsweise ein so genannter Diagnosejob, der von der externen Auswerteeinheit, insbesondere von einem Notebook oder einem Diagnostic Scan Tool oder dergleichen, abgesendet wird und im internen Netzwerk des medizinischen Systems 1 bzw. der Module 10, 20, 30, 40 ausgeführt wird.

Das externe Netzwerk ist beispielsweise als Drahtlosnetzwerk ausgeführt. Zumindest einige der interkommunikations- und netzwerkfähigen Module 10, 20, 30, 40 sind somit dazu ausgebildet, Protokolle nicht nur über das interne Netzwerk bzw. über das interne Bussystem, insbesondere in Echtzeit, zu kommunizieren, sondern sind auch derart ausgeführt, dass Protokolle auf das externe Netzwerk gesendet und von dort empfangen werden können. Zur Kommunikation mit dem externen Netzwerk weisen die Module 10, 20, 30, 40 bzw. das interne Netzwerk bzw. Bussystem BS zumindest entsprechend ausgebildete drahtlose oder drahtgebundene externe Schnittstellen ES auf.

Die Kommunikation mit dem externen Netzwerk ermöglicht das Einspielen von Software und insbesondere so genannter OTA (engl.: Over-the-air) - Updates einzelner Softwarepakete. Diese Softwarepakete können beispielsweise zum Betrieb des modularen medizinischen Systems 1 fungieren und/oder Teile von medizinischer Anwendersoftware darstellen, insbesondere zum Betrieb der Bildgebungseinheit 100 dienen.

Das interne Netzwerk bzw. Bussystem BS weist beispielsweise zumindest einen für Ferndiagnose und/oder Fernwartung eingerichteten Zugangsport in eine Cloud-Anwendung auf. Dies ermöglicht insbesondere einen Abgleich von internen Statusdaten, beispielsweise von Ist-Daten des modularen medizinischen Systems 1 bzw. der Module 10, 20, 30 ,40 "on board", mit externen Normdaten. Bei den externen Normdaten kann es sich beispielsweise um Soll-Daten handeln, die insbesondere in dem externen Netzwerk eines Krankenhauses oder einer ähnlichen medizinischen Einrichtung gespeichert sind. Durch diese Maßnahmen kann eine Fernwartung des medizinischen Systems 1 und deren Komponenten realisiert oder zumindest vorbereitend unterstützt werden und/oder insbesondere die auf der Trägerplattform 3 angeordnete Bildgebungseinrichtung 100 für die prädiktive Instandhaltung (engl.: predictive maintenance) zugänglich gemacht werden. Beispielsweise können Zustandsdaten der Bildgebungseinrichtung 100 aus einer Zentrale abgerufen werden, ohne zeit- und kostenaufwändig einen oder mehrere Servicetechniker in die unmittelbare Nähe der Bildgebungseinrichtung 100 entsenden zu müssen. Dies bringt insbesondere auch Vorteile hinsichtlich der einzuhaltenden Hygienestandards in einer klinischen Umgebung.

Das Basismodul 10 ist maßgeblich für die mechanische Fortbewegung des medizinischen Systems 1 bzw. der ,Trägerplattform 3 vorgesehen. Es weist keine omnidirektionalen Räder, insbesondere keine Mecanum-Räder, auf. Das Fahrwerk 12 des Basismoduls 10 weist eine vergleichsweise einfache Mechanik auf, welche eine Fortbewegung des medizinischen Systems 1 auf konstruktiv einfach gestalteten, abrollenden Abrollkörpern 18 ermöglicht.

Das Basismodul 10 verfügt über eine Vibrationssensorik 11, welche dazu ausgebildet ist, Vibrationen zu erfassen, die bei der Fahrt des medizinischen Systems 1 entstehen. Die Vibrationssensorik 11 umfasst hierzu beispielsweise einen Beschleunigungssensor.

Die Vibrationssensorik 11 des Basismoduls 10 ist dazu ausgebildet, Erschütterungen zu erkennen, die während einer Fahrt auftreten. Anhand der erfassten Vibrationen können Rückschlüsse über die Beschaffenheit des Weges bzw. der Fahrbahn herangezogen gezogen werden.

Wie insbesondere in Figur 2 dargestellt, wird in Ausgestaltungen das Fahrwerk 12 des Basismoduls 10 beim Betrieb des medizinischen Systems 1 von dem Steuerungsmodul 40 mittels geeigneter Fahrsteuerbefehle FB in Abhängigkeit der erfassten Vibrationen angesteuert, beispielsweise derart, dass beim Verlassen eines ebenen Untergrundes bzw. einer ebenen Bodenfläche B das Basismodul 10 automatisch den Fahrbetrieb anhält bzw. stoppt. Auf diese Weise können beispielsweise Beschädigungen an der Unterseite der Trägerplattform 3 und/oder des Basismoduls 10 aufgrund von einer unebenen Untergrundbeschaffenheit vermieden werden.

Das Basismodul 10 leitet beispielsweise die Daten VD aus der Vibrationssensorik 11 über die vorgenannte Buskommunikation an das Umgebungserfassungsmodul 20 weiter. Alternativ dazu werden die aus der Vibrationssensorik 11 stammenden Daten VD direkt mittels des internen Bussystems BS an das Steuerungsmodul 40 weitergegeben.

Darüber hinaus verfügt das Basismodul 10 beispielsweise über eine Beschleunigungssensorik 13, die insbesondere dazu ausgebildet ist, Beschleunigungen zu erfassen, die beim Fahren entstehen. Diese Beschleunigungssensorik 13 umfasst in Ausgestaltungen zumindest einen Beschleunigungssensor, der insbesondere eine andere Bauform aufweist, als der Beschleunigungssensor der vorgenannten Vibrationssensorik 11.

Die Beschleunigungssensorik 13 des Basismoduls 10 ist insbesondere dazu ausgebildet, zu erfassen, wenn eine Fahrt abrupt stoppt oder startet. Dies kann als Maß für etwaige Zusammenstöße des medizinischem Systems 1 mit anderen bewegten oder unbewegten Objekten, wie etwa Personen, Gegenständen, Wänden, Mauern oder ähnlichem, herangezogen werden.

Das Basismodul 10 ist dazu ausgebildet, die Daten BD aus der Beschleunigungssensorik 13 über die vorgenannte Buskommunikation an das Umgebungserfassungsmodul 20 weiterzugeben. Alternativ ist das Basismodul 10 dazu ausgebildet, die von der Beschleunigungssensorik 13 erfassten Daten direkt intern mittels Buskommunikation an das Steuerungsmodul 40 weiterzuleiten.

Das Basismodul 10 weist in Ausgestaltungen eine diskrete elektrische Diagnoseleitung Diag_{A} auf, über die Diagnoseprotokolle, insbesondere eine Zustandsüberwachung des Basismoduls 10, erkannte Zusammenstöße, eine Schlechtwegerkennung oder Ähnliches, an das Steuerungsmodul 40 weitergeben werden können.

In hierzu alternativen Ausgestaltungen ist keine diskrete Diagnoseleitung vorgesehen. Die Diagnoseprotokolle werden in diesem Fall beispielsweise über die vorstehend beschriebene Buskommunikation, also über das Bussystem BS, gesendet und empfangen.

Figur 2 illustriert schematisch den Aufbau und das verfahrenstechnische Zusammenwirken der Module 10, 20, 30, 40 des medizinischen Systems 1. Das Basismodul 10 des dargestellten medizinischen System 1 weist eine diskrete Diagnoseleitung Diag_{A} auf. Bei dem dargestellten und lediglich beispielhaft aufzufassenden medizinischen System 1 wird die über die Beschleunigungssensorik 13 und/oder über die Vibrationssensorik 11 implementierte Schlechtwegkennung von dem Basismodul 10 als qualifizierte Rohdaten QRD mittels Buskommunikation an das Umgebungserfassungsmodul 20 übermittelt und dort weiterqualifiziert.

Wie insbesondere in Figuren 3, 4 und 5 dargestellt, umfasst das Fahrwerk 12 des Basismoduls 10 mehrere unabhängig voneinander steuer- und/oder antreibbare walzen- oder tonnenförmige Abrollkörper 18, auf denen sich das mobile medizinische System 1 fortbewegen kann. Die Abrollkörper 18 sind beispielsweise mittels eines elektrischen Antriebs 14 derart antreibbar, dass die Antriebsachse des elektrischen Antriebs 14 der Rotationsachse R des jeweilig angekoppelten Abrollkörpers 18 entspricht. Auf diese Weise sind somit in Ausgestaltungen Abrollkörper 18 mit Direktantrieb implementiert.

Figuren 3, 4 und 5 illustrieren exemplarisch Fahrwerke 12 mit jeweils acht unabhängig voneinander steuer- und antreibbaren Abrollkörper 18.

Figuren 3 und 4 zeigen beispielhaft ein medizinisches System 1 mit einer als Computertomograph (CT) ausgeführten Bildgebungseinrichtung 100, die über eine mobile Gantry 101 verfügt, in einer perspektivischen Darstellung. Die Abrollkörper 18 sind jeweils paarweise entlang Rotationsachsen R derart angeordnet, dass sie sich bei Bewegung des medizinischen Systems 1 unter Bodenkontakt drehen und statisch stabil auf dem Boden abrollen, ohne zur Seite zu kippen.

Die gezeigten Walzen stellen exemplarisch die vorgenannten tonnenförmigen Abrollkörper 18 dar. Jeder Abrollkörper 18A, 18B, 18C, 18D, 18E, 18F, 18G, 18H ist einzeln antreib- und steuerbar.

Jeder Abrollkörper 18A, 18B, 18C, 18D, 18E, 18F, 18G, 18H weist beispielsweise in seinem geometrischen Inneren einen drehfest mit dem Basismodul 10 verbundenen elektrischen Antrieb 14 auf, der dazu ausgebildet ist, ein Antriebsmoment bereitzustellen. Der elektrische Antrieb 14 kann sowohl entgegen, als auch, bei entsprechender Ansteuerung, im Uhrzeigersinn drehen. Dadurch kann ein Vorwärts- und Rückwärtsdrehen der Abrollkörper 18A, 18B, 18C, 18D, 18E, 18F, 18G, 18H bewirkt werden.

Die Abrollkörper 18A, 18B, 18C, 18D, 18E, 18F, 18G, 18H sind jeweils paarweise entlang jeweils einer Rotationsachse R ausgerichtet, so dass vier unterschiedliche Rotationachsen R gebildet sind. Die Rotationsachsen R sind beispielsweise zueinander rechtwinklig, insbesondere in einem Rechteck oder in einem Quadrat angeordnet, wobei die Rotationsachsen R beispielsweise jeweils parallel zu Seitenflächen des Basismoduls 10 bzw. der Trägerplattform 3 verlaufen. Das Fahrwerk 12 umfasst demnach vier Fahrachsen für eine Bewegung des medizinischen Systems 1 nach vorne, nach hinten, nach rechts und nach links.

Die Abrollkörper 18, 18A, 18B, 18C, 18D, 18E, 18F, 18G, 18H weisen insbesondere in der in Figur 3 gezeigten Ausgestaltung keinen konstanten Durchmesser, sondern sind bombiert ausgebildet, so dass jeweils nur ein Kontaktpunkt mit einer Bodenfläche B gebildet ist, der sich beispielsweise mittig auf dem jeweiligen Abrollkörper 18, 18A, 18B, 18C, 18D, 18E, 18F, 18G, 18H befindet. Eine Bombierung ist in der Wälz-bzw. Lagertechnik eine gezielte fassartige, beispielsweise mittige Verdickung eine Walze bzw. eines Abrollkörpers. Zylinderförmige, nicht bombierte Abrollkörper 18 bilden insbesondere anstelle von Kontaktpunkten Kontaktlinien mit der Bodenfläche B, auf der sich das medizinische System 1 bewegt. Dies resultiert in einer erhöhten Rollreibung. Bombierte Abrollkörper 18, 18A, 18B, 18C, 18D, 18E, 18F, 18G, 18H weisen eine geringere Rollreibung auf und bringen eine verbesserte statische Bestimmung bezüglich der Standsicherheit des Basismoduls 10 mit sich.

Figur 4 zeigt beispielhaft eine Ausführung mit zylindrischen Abrollkörpern 18, 18A, 18B, 18C, 18D, 18E, 18F, 18G, 18H ohne Bombierung.

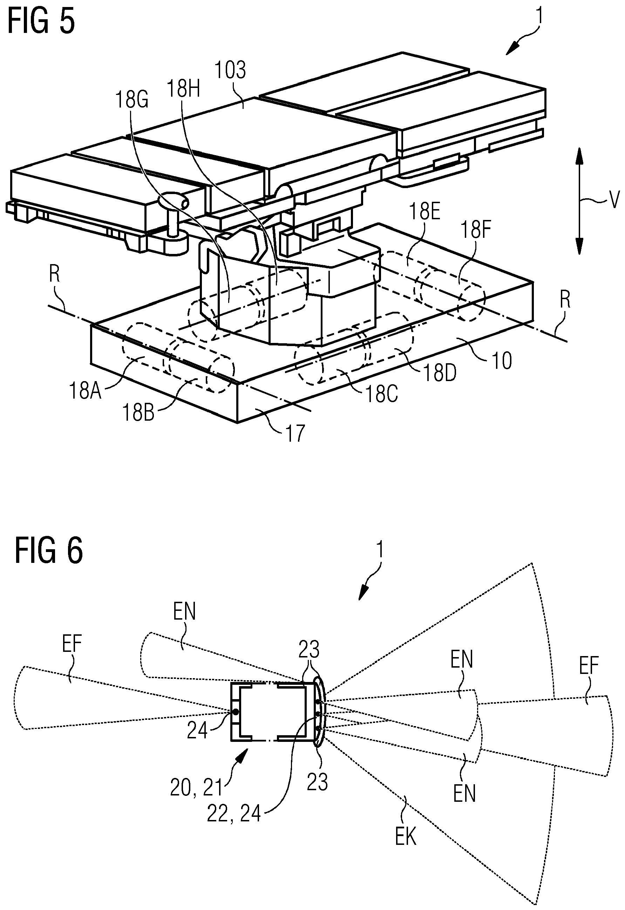

Figur 5 zeigt ein Ausführungsbeispiel des mobilen medizinischen Systems 1, das als medizinische Ausrüstung 102 eine Patientenliege 103 trägt. Das Basismodul 10 mit den unabhängig voneinander steuer- und antreibbaren Abrollkörpern 18, 18A, 18B, 18C, 18D, 18E, 18F, 18G, 18H entspricht im Wesentlichen der bereits mit Bezug auf Figuren 3 und 4 beschriebenen Ausführung. Das Basismodul 10 weist eine im bodennahen Bereich umlaufende Hygieneschürze 17 auf.

Das vorstehend bereits genannte Antriebskonzept mit acht voneinander unabhängig steuer- und antreibbaren Abrollkörpern 18, 18A, 18B, 18C, 18D, 18E, 18F, 18G, 18H ist somit gleichermaßen dazu geeignet, eine Bildgebungseinrichtung 100, insbesondere einen mobilen C-Bogen oder einen Computertomograph mit Gantry 101, oder eine Patientenliege 103 fortzubewegen.

Das medizinische System 1 kann wie folgt bewegt werden: Werden beispielsweise die Abrollkörper 18A, 18B und/oder die Abrollkörper 18E, 18F (vgl. insbesondere Figuren 3 und 4) in eine Drehbewegung versetzt, so fährt das Basismodul 10 in eine Richtung, nach vorne oder nach hinten - je nach Ansteuerung der elektrischen Antriebe 14, die sich beispielsweise im Inneren der Abrollkörper 18A, 18B, 18E, 18F befinden. Werden hingegen die Abrollkörper 18G, 18H und/oder die Abrollkörper 18C, 18D angetrieben (vgl. insbesondere Figuren 3 und 4), so fährt das Basismodul 10 in eine seitliche Richtung, entsprechend der Ansteuerung der elektrischen Antriebe 14. Werden hingegen nur die beiden Walzen 18B und 18E angetrieben (vgl. insbesondere Figuren 3 und 4), so führt das Basismodul 10 einen Linksschwenk aus. Werden hingegen nur die beiden Walzen 18A und 18F angetrieben (vgl. insbesondere Figuren 3 und 4), so führt das Basismodul 10 einen Rechtsschwenk aus.

Insbesondere aus der Darstellung der Figuren 3 und 4 ist unmittelbar ersichtlich, dass sich das Basismodul 10 mit den unabhängig voneinander antreib- und steuerbaren Abrollkörpern 18, 18A, 18B, 18C, 18D, 18E, 18F, 18G, 18H, auf besonderes einfache Art und Weise fortbewegen kann. Insbesondere sind hierzu keine aufwändigen Allseitenräder und insbesondere keine komplexen Mecanum-Räder von Nöten.

In Ausgestaltungen ist bzw. sind ein oder mehrere Abrollkörper 18, 18A, 18B, 18C, 18D, 18E, 18F, 18G, 18H über ein Getriebe von einem elektrischen Antrieb 14 angetrieben. Auf diese Weise ist somit ein indirekter Antrieb implementiert.

Für die Fortbewegung des medizinischen Systems 1 mit Hilfe von unabhängig voneinander antreib- und steuerbarer Abrollkörpern 18, 18A, 18B, 18C, 18D, 18E, 18F, 18G, 18H ist vorgesehen, dass die Abrollkörper 18, 18A, 18B, 18C, 18D, 18E, 18F, 18G, 18H in vertikaler Richtung ein- und ausfahrbar sind. Bei einer Bewegung des Basismoduls 10 in eine Richtung, beispielsweise bei der oben beschriebenen Bewegung nach vorne, werden beispielsweise die Abrollkörper 18A, 18B und/oder die Abrollkörper 18E, 18 F angetrieben. Damit dieses Fahrmanövers nicht negativ von den nicht benötigten Abrollkörper 18C, 18D, 18G, 18H beeinträchtigt wird, werden diese Abrollkörper 18C, 18V, 18G, 18H eingefahren. Auf diese Weise wird zudem der Materialabrieb der Abrollkörper 18C, 18D, 18G, 18H minimiert. Hierzu ist beispielsweise eine Verstelleinrichtung 15, insbesondere eine mechatronische Verstelleinrichtung 15, vorgesehen, die dazu ausgebildet ist, die Abrollkörper 18, 18A, 18B, 18C, 18D, 18E, 18F, 18G, 18H einzeln in vertikaler Richtung V zu verstellen. Die Verstelleinrichtung 15 weist hierzu beispielsweise ein Gestell und einen Aktuator auf. Die Abrollkörper 18, 18A, 18B, 18C, 18D, 18E, 18F, 18G, 18H sind typischerweise federnd gelagert, so dass sich der Federweg beim Einziehen der Abrollkörper 18, 18A, 18B, 18C, 18D, 18E, 18F, 18G, 18H in vertikaler Richtung V verkürzt.

Durch Verstellen der Abrollkörper 18, 18A, 18B, 18C, 18D, 18E, 18F, 18G, 18H in vertikaler Richtung V können die während eines bestimmten Fahrmanövers nicht benötigten Abrollkörper 18, 18A, 18B, 18C, 18D, 18E, 18F, 18G, 18H derart eingezogen werden, dass diese keinen Bodenkontakt mehr aufweisen, so dass Verschleiß vermieden werden kann. Die Verstelleinrichtung 15 ist beispielsweise ein einstellbarer Stoßdämpfer, der als Federwegversteller ausgeführt ist.

Die Verstelleinrichtung 15 ist beispielsweise zum elektrischen, hydraulischen oder pneumatischen Verstellen der Abrollkörper 18, 18A, 18B, 18C, 18D, 18E, 18F, 18G, 18H ausgebildet. Beispielsweise ist die Verstelleinrichtung 15 durch einen regelbareren Gasdruckstoßdämpfer realisiert.

Mit Hilfe der Verstelleinrichtung 15 kann der Abrollkörper 18, 18A, 18B, 18C, 18D, 18E, 18F, 18G, 18H zwischen einer ersten Position, in der der Abrollkörper 18, 18A, 18B, 18C, 18D, 18E, 18F, 18G, 18H Kontakt zur Bodenfläche B hat, und einer zweiten Position, in der der Abrollkörper 18, 18A, 18B, 18C, 18D, 18E, 18F, 18G, 18H von der Bodenfläche B in vertikaler Richtung V beabstandet ist, bewegt werden.

Vorzugsweise ist jeder der unabhängig voneinander steuerbaren Abrollkörper 18, 18A, 18B, 18C, 18D, 18E, 18F, 18G, 18H über eine Verstelleinrichtung 15 in vertikaler Richtung verstellbar. Beispielsweise kann jeder Abrollkörper 18, 18A, 18B, 18C, 18D, 18E, 18F, 18G, 18H über einen separaten, einstellbaren Stoßdämpfer mit dem Basismodul 10 in einer Wirkverbindung stehen.

Das Umgebungserfassungsmodul 20 (auch: Sensorbasis) umfasst eine Umgebungssensorik 21 mit mehreren Gebern und/oder Fühlern und/oder weiteren Sensoren. Die Umgebungssensorik 21 ist zum Erfassen der Umgebung des medizinischen Systems 1 ausgebildet. Das Umgebungserfassungsmodul 20 verfügt über Umgebungssensoren, welche die Umgebung erfassen können, die sich beim Fahren dynamisch ändert. Die Umgebungssensoren der Umgebungssensorik 21 können unterschiedliche Bauformen aufweisen, und sind beispielsweise als Nahfeldradar-Sensoren, Fernfeldradar- Sensoren, LIDAR (engl.: light detection and ranging) Sensoren oder laserscannende Sensorelemente ausgebildet, vorzugsweise in Kombination mit einer Video- und/oder Kamerasensorik. Um eine präzise Nachfelderkennung zu realisieren, sind beispielsweise alternativ oder zusätzlich Ultraschallsensoren vorgesehen, die am Umgebungserfassungsmodul 20 angeordnet sind oder zumindest mit dem Umgebungserfassungsmodul 20 elektrisch verbunden sind.

Die Umgebungssensorik 21 des Umgebungserfassungsmoduls 20 ist durch das Zusammenspiel von mehreren derartigen Sensoren und/oder Sensorelementen in der Lage, sowohl dynamisch bewegte als auch stationäre Personen und Gegenstände, als Umgebungsinformationen bzw. Rohdaten RD zu erfassen. Diese Rohdaten RD (vgl. insbesondere Figur 2) werden intern an das Umgebungserfassungsmodul 20 weitergegeben bzw. dort weiterverarbeitet. Innerhalb des Umgebungserfassungsmoduls 20 wird beispielsweise, wie bereits beschrieben, ein Betriebssystem (OS) betrieben. Das Betriebssystem ermöglicht beispielsweise das Ablaufen von Bilderkennungssoftware innerhalb des Umgebungserfassungsmoduls 20. Mittels dieser Bilderkennungssoftware erfolgt eine Qualifizierung der Rohdaten, d. h. aus der dynamischen und stationären Erfassung von Objekten, insbesondere von Personen und Gegenständen, in der Umgebung des medizinischen Systems 1 resultiert die tatsächliche Erkennung von Personen und Gegenständen. Die erkannten Personen und Gegenständen können sodann als qualifizierte Umgebungsdaten QUD an das Steuerungsmodul 40 weitergeben werden, insbesondere derart, dass das im Steuerungsmodul 40 ein virtuelles Abbild der Umgebung erstellt werden kann, mit dem Ziel, eine kollisionsfreie Wegplanung für den Fahrweg und die Fahrrichtung des medizinischen Systems 1 bereitzustellen.

Darüber hinaus weist das medizinische System 1 in Ausgestaltungen (vgl. insbesondere Figur 2) eine diskrete elektrische Diagnoseleitung Diagₛ auf, über welcher Diagnoseprotokolle (z.B. Fehlermustererkennung, Kollisionserkennung) an das Steuerungsmodul 40 (auch: Steuerungsbasis) übertragen werden können.

In hierzu alternativen Ausgestaltungen ist keine diskrete Diagnoseleitung vorgesehen. Die Diagnoseprotokolle werden in diesem Fall beispielsweise über die vorstehend beschriebene Buskommunikation, also über das Bussystem BS, gesendet und empfangen.

Figur 6 zeigt ein mögliches Ausführungsbeispiel eines medizinischen Systems 1 mit einem eine Umgebungssensorik 21 aufweisenden Umgebungserfassungsmodul 20. Die Umgebungssensorik 21 umfasst in Fahrtrichtung nach vorne gerichtete Kameras 22, die einen im Wesentlichen kegelförmigen Erfassungsbereich EK aufweisen. Die Umgebungssensorik 21 umfasst ferner in Fahrtrichtung nach vorne und entgegen der Fahrtrichtung nach hinten gerichtete Nahfeldradar-Sensoren 23 und Fernfeldradar-Sensoren 24. Die Nahfeldradar-Sensoren 23 weisen im Wesentlichen kegelförmige Erfassungsbereiche EN auf. Entsprechend weisen die Fernfeldradar-Sensoren 24 im Wesentlichen kegelförmige Erfassungsbereiche EF mit größerer Reichweite auf. Die Nahfeldradar-Sensoren 23 sind beispielsweise 24 GHz Radarsensoren. Die Fernfeldradar-Sensoren 24 sind beispielsweise 77 GHz Radarsensoren.

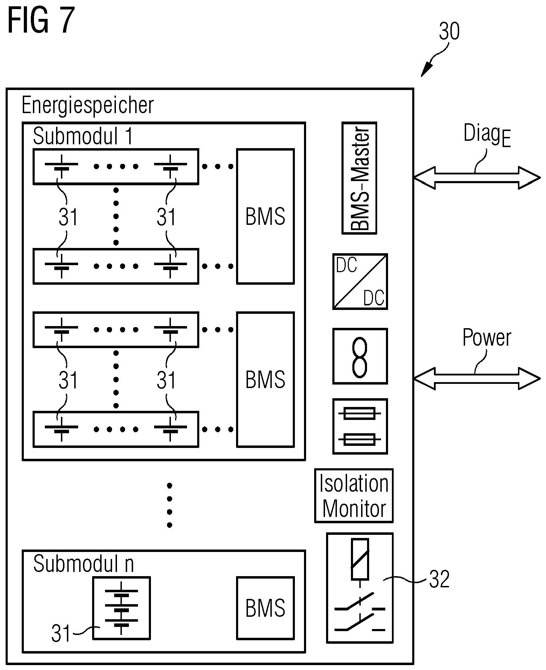

Das, insbesondere optional vorgesehene, Energiespeichermodul 30 (auch: Versorgungsbasis) ist in einer möglichen Ausführungsform in Figur 7 dargestellt. Das Energiespeichermodul 30 dient insbesondere dazu, eine Autonomie bzw. Autarkie von stationären Versorgungsnetzen sicherzustellen.

Das Energiespeichermodul 30 stellt die Strom- und Spannungsversorgung für die Module 10, 20, 30 bereit und weist hierfür mehrere Energiezellen 31 zum Speichern von elektrischer Energie auf. Das Energiespeichermodul 30 ist zur Stromversorgung mit den Module 10, 20, 30 und der gegebenenfalls optional vorhandenen Bildgebungseinrichtung 100 über elektrische Leitungen verbunden L. Die Energiezellen 31 sind beispielsweise als Batterien oder Akkumulatoren ausgeführt und weisen ferner ein internes Steuer- und Regelungssystem, ein so genanntes Batterie- oder Akkumulatormanagementsystem BMS auf. Das Batteriemanagementsystem BMS ist insbesondere dazu ausgebildet, den Ladezustand (engl.: state-of-charge, SOC) und den Verfügbarkeitszustand (engl.: state-of-health, SOH) der Energiezellen 31 ermitteln, bevorzugt in Echtzeit, so dass stets interne und aktuelle Informationen über eine Restlaufzeit des Energiespeichermoduls 30 verfügbar sind.

Der Ladezustand kann, wie insbesondere in Figur 2 dargestellt, bei einem Verfahren zum Betrieb des medizinischen Systems 1 dazu dienen, dem Steuerungsmodul 40 eine Echtzeitinformation der verbleibenden Kapazität der Energiezellen 31 des Energiespeichermoduls 30 zu übermitteln. Die Kommunikation des Energiespeichermoduls 30 mit dem Steuerungsmodul 40 erfolgt über eine dedizierte bzw. diskrete Diagnoseleitung Diag_{E} oder über die bereits beschriebene Buskommunikation, die vom Bussystem BS bereitgestellt wird.

Der Verfügbarkeitszustand ist ein Maß für die Fähigkeit der Energiezellen 31 "on board", ihre spezifizierte Leistung zu liefern. Dies ist insbesondere für die Beurteilung der Bereitschaft von Notstromgeräten wichtig und ein Indikator dafür, ob Wartungsmaßnahmen, insbesondere im Rahmen der vorgenannten Predictive Maintenance erforderlich sind.

Das Batterie-Management-System BMS ist in Ausgestaltungen dazu ausgebildet, ein Verfahren zum Zellenschutz zu implementieren. Dieser Zellenschutz dient zur Einhaltung der Auslegungsgrenzen der Energiezellen 31. Ein Betrieb einer Energiezelle 31 außerhalb der vorgegebenen Auslegungsgrenzen führt im Allgemeinen zum Ausfall der entsprechenden Energiezelle 31 und somit zu weiteren Kosten für einen erforderlichen Batteriewechsel.

Das Energiespeichermodul 30 weist in Ausgestaltungen weitere Kommunikationsschnittstellen auf, die einem Benutzer oder dem Steuerungsmodul 40 Zugriff ermöglichen, insbesondere um Steuerparameter des Batteriemanagementsystem BMS zu ändern oder eine Diagnose bzw. einen Test des Energiespeichermoduls 30 durchzuführen.

Über die diskrete elektrische Diagnoseleitung Diag_{E} können Diagnoseprotokolle (z.B. Ladezustand, Verfügbarkeitszustand, Zellenschutz, vorhandene Fehler) beim Betrieb des medizinischen Systems 1 an das Steuerungsmodul 40 weitergeben werden. In hierzu alternativen Ausgestaltungen ist keine diskrete Diagnoseleitung vorgesehen. Die Diagnoseprotokolle werden in diesem Fall beispielsweise über die vorstehend beschriebene Buskommunikation bzw. über das interne Bussystem BS gesendet und empfangen.

Das Energiespeichermodul 30 umfasst in Ausgestaltungen beispielsweise Energiezellen 31, die als Nickel-Metallhydrit (NiMH) Batterien ausgeführt sind. Alternativ dazu sind die Energiezellen 31 beispielsweise als AGM-Batterien, als Lithium-Ionen-Batterien (LiB) oder als Lithium-Polymer-Akkumulatoren (LiPo) ausgeführt. Die Energiezellen 31 sind zur Leistungs- und Energieskalierung beispielsweise in Parallel- und Serienschaltung angeordnet und in vorteilhafter Weise in Zellpakete bzw. Module und Submodule aufgeteilt.

Um möglichst viele unterschiedliche Lastszenarien bedienen zu können, ist in Ausgestaltungen eine Zusammenschaltung verschiedener Energiezellen 31 vorgesehen. Dies ist in Figur 7 durch den Schalter 32 schematisch illustriert.

Das Energiespeichermodul 30 umfasst in Ausgestaltungen eine Brennstoffzelle, insbesondere eine mit Methanol, Butan oder Ähnlichem betreibbare Brennstoffzelle. Entsprechend ist in diesen Ausgestaltungen des im Energiespeichermoduls 30 vorzugsweise eine Steuerung und/oder ein Energiemanagementsystem der Brennstoffzelle implementiert. Eine derartiges Energiespeichermodul 30 ist insbesondere dazu ausgebildet, energieautark in einer Feldumgebung wie etwa einem Feldlazarett in einem Kriegs- oder Katastrophengebiet eingesetzt zu werden. Energieautarkie kann in diesen Fällen entscheidende Vorteile hinsichtlich der Verfügbarkeit des medizinischen Systems 1 bieten.

Das Energiespeichermodul 30 weist in Ausgestaltungen neben dem Batteriemanagementsystem BMS Alternativen zu handelsüblichen Akkumulatoren oder Batterien mit einer hohen Energiedichte auf. Beispielsweise weist das Energiespeichermodul 30 eine Lithium-Luft Batterie als Energiespeicher auf. Derartige Energiespeicher können eine 5 bis 10-fach höhere Energiedichte als herkömmliche Lithium-Ionen-Batterien aufweisen. In anderen Ausführungen weist das Energiespeichermodul 30 beispielsweise einen Akkumulator auf Siliziumbasis auf, der Anoden aus reinem Silizium umfasst. Gegenüber der gängigen Lithium-Ionen-Technik kann ein derartiger Energiespeicher beispielsweise eine zehnfach erhöhte Energiedichte aufweisen. In anderen Ausführungen weist das Energiespeichermodul 30 beispielsweise so genannte Lithium-Eisenphosphat- Akkumulatoren (LiFeP-Akkumulatoren) oder Lithium-Titanat-Akkumulatoren (Li-Ti- Akkumulatoren) auf. Da das Titanat nicht mit Oxiden aus der negativen Elektrode reagieren kann, kann bei einer derartigen Ausführung das thermische Durchgehen des Akkumulators verhindert werden, u. U. auch bei Vorliegen von mechanischen Schäden. Ein Lithium-Titanat-Akkumulator kann in vorteilhafter Weise bei tiefen Temperaturen, beispielsweise in einem Temperaturbereich von -40 °C bis +55 °C betrieben werden.

Das Energiespeichermodul 30 umfasst in Ausgestaltungen eine so genannte Redox-Flow-Batterie (RFB), etwa in Form eines Vanadium-Redox-Akkumulators, eines Natriumbromid-Redox-Akkumulators oder eines Zink-Brom-Akkumulators, als Energiespeicher.

Das Energiespeichermodul 30 umfasst in Ausgestaltungen eine so genannte Feststoffbatterie (auch: Festkörperakkumulator) als Energiespeicher. Derartige Ausführungen weisen eine erhöhte Sicherheit auf, da bei derartigen Feststoffbatterien sowohl beide Elektroden und auch der Elektrolyt aus festem Material bestehen. Insbesondere im Vergleich zu herkömmlichen Lithium-Ionen-Akkumulatoren ist eine solche Feststoffbatterie nur relativ schwer entflammbar. In Bezug auf eine Zulassung des medizinischen Systems 1 in einer klinischen Umgebung wie etwa einem Krankenhaus, einem Medical Center, einer Praxis oder Ähnlichem kann die Brennbarkeit des verwendeten Energiespeichers eine wichtige Rolle spielen.

Das Steuerungsmodul 40 (auch: Kontrollmodul, Steuerungsbasis) ist dazu ausgebildet, Daten bzw. Informationen aus dem Umgebungserfassungsmodul 20 intern zu verarbeiten und/oder zu qualifizieren, in Fahrsteuerbefehle FB umzuwandeln und an das Basismodul 10 weiterzugeben. Das Steuerungsmodul 40 ist ferner für die Erfassung, Verarbeitung und Realisierung sowohl von Benutzerinteraktionen als auch für die Systemüberwachung, Kalibrierung, Interkonnektivität mit klinischen IT-Systemen, wie etwa einem Hospitalinformationssystem (HIS), einem Laborinformationssystem (LIS) oder einem so genannten Picture Archiving or Communication System (PACS) und Datensicherheitsüberwachung verantwortlich.

Zur Erfassung von Benutzerinteraktionen weist das Steuerungsmodul 40 eine Benutzerschnittstelle 41, die beispielsweise Eingabegeräte, wie etwa einen Touchscreen, eine Tastatur oder eine Einrichtung umfasst, die zur Erfassung und Erkennung von Gesten des Benutzers und/oder Sprachkommandos ausgebildet ist.

Das Steuerungsmodul 40 ist in Ausgestaltung beispielsweise dazu ausgebildet, folgende Funktionen zu implementieren:

| **Funktion** | **Beispiel** |
|---|---|
| Benutzerinteraktionen | Motorisierte Unterstützung von Bewegungen |
| | Sprach- und Gestensteuerungen |
| Systemüberwachung | Verschleißvorhersage (Predictive Maintenance) |
| | Zustandsüberwachung (Condition Monitoring) Selbstdiagnose und Fehleranzeige |
| Kalibrierung | (Semi-) autonome in-situ Kalibrierroutinen (z. B. Lokalisierung- und Navigationsaufgaben, Verschleißerkennung) |
| Datensicherheitsüberwachung | Datensicherheit, Datenintegrität (z. B. Prüfsummen) |
| | Datenschutz (z. B. PKI Methoden) |

Das Steuerungsmodul 40 umfasst eine leistungsfähige Regel- und Steuerungselektronik, um den teil-autonomen oder autonomen Betrieb des medizinischen Systems 1 bzw. der Trägerplattform 3 zu ermöglichen. Innerhalb des Steuerungsmoduls 40 ist in Ausgestaltungen, wie beispielsweise auch in den übrigen Modulen 10, 20, 30, ein Betriebssystem (OS) installiert. Das Betriebssystem des Steuerungsmoduls 40 ermöglicht die Installation von weiterer Software. In Ausgestaltungen ist eine Wegplanungs- bzw. Navigationssoftware innerhalb der Steuerungsmoduls 40 implementiert.

Wie insbesondere in Figur 2 illustriert, erfolgt die Wegplanung bzw. Navigation bei einem Verfahren zum Betrieb des medizinischen Systems 1 in Abhängigkeit der qualifizierten Umgebungsdaten QUD, insbesondere in Abhängigkeit der sensorisch erfassten und erkannten Personen und Gegenstände beispielsweise derart, dass im Steuerungsmodul 40 ein virtuelles Abbild der Umgebung erstellt wird. Das virtuelles Abbild der Umgebung dient dem Ziel, eine kollisionsfreie Wegplanung für den Fahrweg und die Fahrtrichtung des mobilen medizinischen Systems 1 bzw. der Trägerplattform 3 bereit zu stellen.

Das Steuerungsmodul 40 übermittelt im Betrieb beispielsweise in Abhängigkeit des virtuellen Abbildes der Umgebung Fahrsteuerbefehle FB an das Basismodul 10 zur Ansteuerung der Antriebsmittel 16. Das Steuerungsmodul 40 ist dazu ausgebildet, die Fortbewegung des medizinischen Systems 1 zu regeln und dabei insbesondere die Umgebung und/oder den Energiezustand des Energiespeichermoduls 30 zu berücksichtigen, welcher beispielsweise wie vorgenannt durch das Batteriemanagementsystem BMS des Energiespeichermoduls 30 ermittelt und über das Bussystem BS dem Steuerungsmodul 40 zur Verfügung gestellt wird.

Einige beispielhafte Fahrsteuerbefehle FB können eine Vorwärts-, Rückwärts-, Linksschwenk- (45 Grad), Rechtsschwenk-(45 Grad), Steuerbord- (Fahrtrichtung nach links), Backbord-(Fahrtrichtung nach rechts) Bewegung betreffen.

In Ausgestaltungen weist das Steuerungsmodul 40 eine so genannte Voice Assistance Funktionalität auf und insbesondere eine dafür ausgelegte Software sowie eine Benutzerschnittstelle 41, die zum Erfassen von menschlicher Sprache ausgebildet ist. Die Benutzerschnittstelle 41 weist hierfür beispielsweise Mikrofone oder Ähnliches auf. Die Spracherkennungssoftware ist zur Erkennen zumindest einer Sprache ausgebildet.

Mittels einer solchen Voice Assistance Software ist es möglich, dass Personen, wie etwa Ärzte, Klinikpersonal, medizinisch-technische Assistenten (MTA), Sprachkommandos abgeben, welche die Voice Assistance Funktion erfassen und als solche erkennen kann. Die Sprachkommandos können beispielsweise zu den vorgenannten Fahrsteuerbefehlen FB identisch sein und beispielsweise darin bestehen, dass Worte wie "vorwärts", "linksschwenk 45" (grad), "rechtsschwenk 45" (grad), "rückwärts", "links" (Steuerbord), "rechts" (Backbord) laut ausgesprochen werden. Die Ausführung eines der Fahrsteuerbefehle FB durch das Basismodul 10 beinhaltet beispielsweise das Verstellen zumindest eines der Abrollkörper 18, 18A, 18B, 18C, 18D, 18E, 18F, 18G, 18H in vertikaler Richtung V beinhaltet, so dass das entsprechende Fahrmanöver durchgeführt werden kann.

Die Sprachkommandos oder sprachlichen Kommandos werden beim Betrieb des medizinischen Systems 1 beispielsweise innerhalb des Steuerungsmoduls 40 priorisiert gegenüber den intern anhand des virtuellen Abbildes erzeugten Fahrsteuerbefehlen FB abgearbeitet, so dass im Ergebnis der Benutzer (MTA, Arzt, Klinikpersonal) die Bewegung des medizinischen Systems 1 in Ausgestaltungen vollständig anhand von sprachlichen Kommandos steuern kann, anstatt auf die seitens des Steuerungsmoduls 40 generierten Fahrsteuerbefehle FB vertrauen zu müssen.

Im Kontext der hier vorliegenden Beschreibung kann die Steuerung über sprachliche Kommandos als "teil-autonomer" Betrieb der medizinischen Systems 1 angesehen werden. Vorteilhafterweise verfügt das Steuerungsmodul 40 über ein Steuergeräte-Gateway, d.h. über eine qualifizierte Daten-und/oder Kommunikationsschnittstelle für die Netzwerkkommunikation zwischen dem internen Bussystem BS und einem externen Netzwerk, beispielsweise einem Drahtlosnetzwerk oder einer "Cloud". Aus dem externen Netzwerk können beispielsweise Over-the-air-Updates einzelner Softwarepakete heruntergeladen werden. Auf diese Art und Weise kann auf vorteilhafte Weise das Einspielen von zukünftigen Softwareupdates als Dienstleistungen angeboten werden.

Die Erfindung betrifft auch das vorteilhafte technische Zusammenwirken und Steuerverfahren der vorgenannten Module 10, 20, 30, 40. Das medizinische System 1 umfasst das Basismodul 10, das Umgebungserfassungsmodul 20 und das Steuerungsmodul 40, optional zusätzlich das Energiespeichermodul 30, um eine autonome oder zumindest teil-autonome Bewegung des medizinischen Systems 1 von einem Ort (beispielsweise einem Krankenhaus, einem Medical Center, einer Praxis, einem Feldlazarett, etc.) zu einem weiteren/nächsten Ort, der vom ersten Ort verschieden ist, zu ermöglichen.

Obwohl die Erfindung im Detail mit Bezug auf die bevorzugten Ausführungsbeispiele näher illustriert und beschrieben wurde, so ist die Erfindung nicht hierdurch eingeschränkt. Andere Variationen und Kombinationen können vom Fachmann hieraus abgeleitet werden, ohne vom wesentlichen Gedanken der Erfindung abzuweichen.

## Patentansprüche

1. Basismodul (10) für ein mobiles medizinisches System (1), welches dazu ausgebildet ist, Wegstrecken autonom oder semi-autonom zurückzulegen, mit einem mehrere Abrollkörper (18, 18A, 18B, 18C, 18D, 18E, 18F, 18G, 18H) aufweisenden Fahrwerk (12), wobei die Abrollkörper (18, 18A, 18B, 18C, 18D, 18E, 18F, 18G, 18H) um Rotationsachsen (R) drehbar gelagert sind und mittels Verstelleinrichtungen (15) in vertikaler Richtung (V) derart verstellbar sind, dass jeder Abrollkörper (18, 18A, 18B, 18C, 18D, 18E, 18F, 18G, 18H) zwischen einer ersten Position, in der der jeweilige Abrollkörper (18, 18A, 18B, 18C, 18D, 18E, 18F, 18G, 18H) in Kontakt zu einer Bodenfläche (B) steht, und einer zweiten Position, in der der jeweilige Abrollkörper (18, 18A, 18B, 18C, 18D, 18E, 18F, 18G, 18H) von der Bodenfläche (B) beabstandet ist, bewegbar ist.

2. Basismodul (10) nach Anspruch 1, wobei zumindest zwei Rotationsachsen (R) der Abrollkörper (18, 18A, 18B, 18C, 18D, 18E, 18F, 18G, 18H) zueinander in einer Anordnung angeordnet sind, die von einer parallelen oder antiparallelen Anordnung abweicht.

3. Basismodul (10) nach Anspruch 1 oder 2, wobei zumindest zwei Rotationsachsen (R) der Abrollkörper (18, 18A, 18B, 18C, 18D, 18E, 18F, 18G, 18H) zueinander in einem stumpfen, insbesondere rechten, Winkel angeordnet sind.

4. Basismodul (10) nach einem der vorhergehenden Ansprüche, wobei die Rotationsachsen (R) der Abrollkörper (18, 18A, 18B, 18C, 18D, 18E, 18F, 18G, 18H) zumindest eine umlaufende, insbesondere rechteckige oder quadratische, Anordnung bilden.

5. Basismodul (10) nach einem der vorhergehenden Ansprüche, wobei die Abrollkörper (18, 18A, 18B, 18C, 18D, 18E, 18F, 18G, 18H) jeweils paarweise um die Rotationsachsen (R) drehbar gelagert sind.

6. Basismodul (10) nach einem der vorhergehenden Ansprüche, wobei die Verstelleinrichtung (15) als einstellbarer Stoßdämpfer, insbesondere Gasdruckstoßdämpfer, ausgeführt ist.

7. Basismodul (10) nach einem der vorhergehenden Ansprüche, wobei die Abrollkörper (18, 18A, 18B, 18C, 18D, 18E, 18F, 18G, 18H) im Wesentlich zylindrisch ausgeführt sind.

8. Basismodul (10) nach einem der vorhergehenden Ansprüche, wobei die Abrollkörper (18, 18A, 18B, 18C, 18D, 18E, 18F, 18G, 18H) eine Bombierung aufweisen.

9. Basismodul (10) nach einem der vorhergehenden Ansprüche, mit einer Vibrationssensorik (11) zur Erfassung von Vibrationen, insbesondere von während der Fahrt auftretenden Vibrationen.

10. Basismodul (10) nach einem der vorhergehenden Ansprüche, mit einer Beschleunigungssensorik (13) zur Erfassung von Beschleunigungen, insbesondere Kollisionen.

11. Medizinisches System (1), welches dazu ausgebildet ist, Wegstrecken autonom oder semi-autonom zurückzulegen, mit
- einem Basismodul (10) nach einem der vorhergehenden Ansprüche,
- einem Umgebungserfassungsmodul (20), welches eine zur Erfassung der Umgebung des medizinischen Systems ausgebildete Umgebungssensorik aufweist,
- ein Steuerungsmodul (40) zur Steuerung der Fortbewegung des Basismoduls (10) unter Berücksichtigung der mittels des Umgebungserfassungsmoduls (20) sensorisch erfassten Umgebung des medizinischen Systems (1).

12. Medizinisches System (1) nach Anspruch 11, mit einem Energiespeichermodul 30 zur Versorgung des medizinischen Systems (1) mit Energie, wobei das Steuerungsmodul (40) dazu ausgebildet ist, die Fortbewegung des Basismoduls (10) unter Berücksichtigung des Energie- bzw. Ladezustands des Energiespeichermoduls (30) zu steuern.

13. Medizinisches System (1) nach Anspruch 11 oder 12, wobei das Steuerungsmodul (40) eine Benutzerschnittstelle (41) aufweist und zur Sprachsteuerung und/oder Gestensteuerung ausgebildet ist.

14. Medizinisches System (1) nach einem der Ansprüche 11 bis 13, welches dazu ausgebildet ist, eine Bildgebungseinrichtung (100), medizinische Ausrüstung (102), insbesondere eine Patientenliege (103) und/oder eine Last (104) zu tragen und fortzubewegen.

15. Verfahren zum Betrieb eines medizinischen Systems (1) gemäß einem der Ansprüche 11 bis 14, wobei das Steuerungsmodul (40) zur Steuerung der Fortbewegung Fahrsteuerbefehle (FB) generiert und zur Ausführung an das Basismodul (10) übermittelt, wobei die Ausführung zumindest eines der Fahrsteuerbefehle (FB) durch das Basismodul (10) das Verstellen zumindest eines der Abrollkörper (18, 18A, 18B, 18C, 18D, 18E, 18F, 18G, 18H) in vertikaler Richtung (V) beinhaltet.

16. Verfahren nach Anspruch 15, wobei die Generierung der Fahrsteuerbefehle (FB) in Abhängigkeit von Daten (VD) einer Vibrationssensorik (11) erfolgt, die insbesondere indikativ für während der Fahrt auftretende Vibrationen sind.

17. Verfahren nach Anspruch 15 oder 16, wobei die Generierung der Fahrsteuerbefehle (FB) in Abhängigkeit von Daten (VD) einer Beschleunigungssensorik (13) erfolgt, die insbesondere indikativ für während der Fahrt auftretende Beschleunigungen sind.

18. Verfahren nach einem der Ansprüche 15 bis 17, wobei die Generierung der Fahrsteuerbefehle (FB) in Abhängigkeit von qualifizierten Umgebungsdaten (QUD) erfolgt, die insbesondere indikativ für das Umfeld des medizinischen Systems (1) sind.

19. Verfahren nach einem der Ansprüche 15 bis 18, wobei die Generierung der Fahrsteuerbefehle (FB) in Abhängigkeit von Diagnoseprotokollen eines Batteriemanagementsystems (BMS) erfolgt, die insbesondere indikativ für einen Ladezustand und/oder einen Verfügbarkeitszustand eines Energiespeichers sind.
